(19) Europäisches Patentamt
European Patent Office
Office européen des brevets

(11) Publication number: **0 031 548**
**B1**

(12) # EUROPEAN PATENT SPECIFICATION

(45) Date of publication of patent specification: **23.03.83**

(51) Int. Cl.³: **C 07 D 285/06**

(21) Application number: **80107981.5**

(22) Date of filing: **17.12.80**

(54) **Process for preparing 5-amino-1,2,3-thiadiazoles.**

(30) Priority: **27.12.79 JP 169292/79**
**07.10.80 JP 139364/80**
**07.10.80 JP 139365/80**

(43) Date of publication of application:
**08.07.81 Bulletin 81/27**

(45) Publication of the grant of the patent:
**23.03.83 Bulletin 83/12**

(84) Designated Contracting States:
**BE CH FR GB IT LI NL**

(56) References cited:
DE - A - 2 636 994
Patents Abstracts of Japan Vol. 1, No. 106, 19 September 1977 page 2419C77
Patents Abstracts of Japan Vol. 1, No. 10, 18 March 1977 page 419C76
CHEMISCHE BERICHTE, Vol. 99, No. 5, 1966 Weinheim/Bergstr.
J. GOERDELER et al. "Über 5-Amino-1.2.3-thiadiazole" pages 1618 to 1631

(73) Proprietor: **Ube Industries, Ltd.**
**12-32, Nishihonmachi 1-chome**
**Ube-shi, Yamaguchi-ken (JP)**

(72) Inventor: **Nakai, Mamoru**
**Nishino-ushiro Osawa Nishikiwa**
**Ube-shi Yamaguchi-ken (JP)**
Inventor: **Harada, Katsumasa**
**3446, Oaza Higashisue**
**Ubeshi Yamaguchi-ken (JP)**
Inventor: **Mori, Yoshikatsu**
**106, Oaza Ariho**
**Onoda-shi Yamaguchi-ken (JP)**

(74) Representative: **Hansen, Bernd, Dr.rer.nat. et al,**
**Hoffmann . Eitle & Partner Patentanwälte**
**Arabellastrasse 4**
**D-8000 München 81 (DE)**

**EP 0 031 548 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing 5-amino-1,2,3-thiadiazoles

This invention relates to a novel process for preparing 5-amino-1,2,3-thiadiazoles.

5-Amino-1,2,3-thiadiazoles have been utilized as raw materials for pharmaceuticals, agricultural chemicals and the like.

Conventionally, there have been proposed, as the methods for preparing 5-amino-1,2,3-thiadiazoles, a method in which chloroaldehyde ethoxycarbonylhydrazine is subjected to reaction with thionyl chloride followed by amination of the resulting reaction product (German Offenlegungsschrift No. P26 36 994); a method in which acetyl isothiocyanate is subjected to reaction with diazomethane [Chem. Ber. *99*, 1618—31 (1966)]: and so on.

However, these conventional methods have problems in that the starting materials are expensive and tedious processes are required for preparing the starting materials, and hence are not necessarily advantageous from the economical standpoint.

As a result of extensive studies to establish an industrially advantageous process for preparing 5-amino-1,2,3-thiadiazoles, the present inventors have found that 5-amino-1,2,3-thiadiazoles can be prepared industrially by bringing a diazoacetonitrile into contact with hydrogen sulfide in the presence of a base or with a salt of hydrogen sulfide, in a solvent, and accomplished this invention.

The reaction according to this invention proceeds in accordance with the following equation.

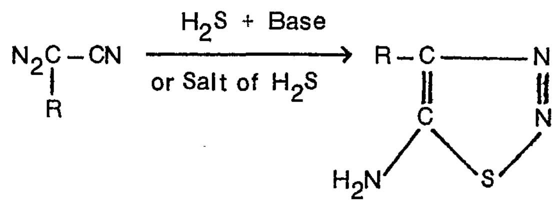


wherein R represents a hydrogen atom, a lower alkyl group or an aryl group.

The lower alkyl group represented by R in the above equation includes an alkyl group having 1 to 6 carbon atoms such as methyl, ethyl, propyl, butyl, pentyl and hexyl.

The aryl group represented by R in the above equation includes phenyl, p-tolyl, p-ethylphenyl and naphthyl.

The reaction can be carried out by a simple procedure, for example, by dissolving a diazoacetonitrile in a solvent and blowing hydrogen sulfide into the solution in the presence of a base or introducing a salt of hydrogen sulfide into the solution.

The diazoacetonitriles which are the starting materials in this invention are substances being difficult to be handled because of their explosiveness or instability. However, these substances are entirely safe in such a solvent as ether, methylene chloride, etc.

The diazoacetonitriles can easily be prepared and obtained by subjecting an aminoacetonitrile to reaction with sodium nitrite, followed by extraction with ether, methylene chloride, etc. The solents used herein are also suitable as the solvents for the reaction according to this invention. Accordingly, in the present invention, the thus obtained diazoacetonitrile are used in situ in a solution without being isolated so that there is no disadvantage such as dangerousness or difficulty in handling.

The aminoacetonitriles are precursors for glycine, which is commercially available in large amount, is inexpensive and can easily be available. Accordingly, the process according to this invention has also advantages in that the starting materials are less expensive and that the method for preparing the starting material is more simple as compared with the known processes.

As the solvent to be used in the present invention, any solvent can be useful as long as it is inert in the reaction and can dissolve the diazoacetonitrile. For example, there may be mentioned an aliphatic hydrocarbon such as n-hexane, n-heptane, etc.; an alicyclic hydrocarbon such as cyclohexane etc.; a halogenated hydrocarbon such as chloroform, methylene chloride, carbon tetrachloride, trichloroethylene, 1,2-dichloroethylene, 1,2-dichloroethane, etc.; an ether such as diethyl ether, dioxane, methyl cellosolve, ethyl cellosolve, etc.; an alcohol such as methanol, ethanol, etc.; and water. These solvents may usually be used alone and may also be used in combination. Industrially however, there may advantageously be employed the solvent such a solvent as methylene chloride, ether, chloroform or n-hexane which are used for the extraction when a diazoacetonitrile is synthesized.

In cases where hydrogen sulfide is used, a base must be used in combination therewith.

As the base to be used in this invention, there may be employed either organic or inorganic base.

As the organic bases, there may be effectively employed an amine series compounds, for example, methyl amine, ethyl amine, n-propyl amine, isopropyl amine, n-butyl amine, dimethyl amine, diethyl amine, dibutyl amine, trimethyl amine, triethyl amine, tributyl amine, allyl amine, α-ethoxyethyl amine, pyridine, ethylenediamine, hexamethylenediamine, hexamethyleneimine, hexamethylenetetramine, hydroxyl amine and the like.

The amount of the organic base to be employed is not less than 0.1 mole, preferably 0.5 to 5 moles, per one mole of the starting diazoacetonitrile.

As the inorganic base, there may effectively be employed an alkali metal hydroxide such as sodium hydroxide, potassium hydroxide, etc.; an alkaline earth metal hydroxide such as calcium hydroxide, barium hydroxide, etc.; an alkali metal carbonate such as sodium carbonate, potassium carbonate, etc.; an alkoxide such as sodium methoxide, sodium ethoxide, etc.; a metal amide such as sodium amide, potassium amide, etc.; ammonia; hydrazine; and so on. It is desirable to dissolve these inorganic bases in solvents as mentioned above before use. In view of the yield of the desired product as well as economy, these inorganic bases may be used in an amount of 0.05 gram equivalent or more, preferably in the range from 0.1 to 0.5 gram equivalent, per mole of the starting diazoacetonitriles.

In cases where hydrogen sulfide is used, hydrogen sulfide is blown into the system thus maintained over 0.1 to 2 hours so that the amount may be substantially equimolar to that of the diazoacetonitrile.

In the present invention, 5-amino-1,2,3-thiadiazoles may also be obtained by contacting a diazoacetonitrile with a salt of hydrogen sulfide. As the salt of hydrogen sulfide to be used in the present invention, there may be mentioned either an organic salt or an inorganic salt.

As the organic salt of hydrogen sulfide, there may effectively be used a salt between hydrogen sulfide and an amine type organic base such as methyl amine, ethyl amine, n-propyl amine, isopropyl amine, n-butyl amine, dimethyl amine, diethyl amine, trimethyl amine, triethyl amine, tributyl amine, allyl amine, 2-ethoxyethyl amine, pyridine, ethylene diamine, hexamethylene diamine, hexamethylene imine, hexamethylene tetramine, hydroxyl amine, etc. These organic salts of hydrogen sulfide may readily be prepared by blowing hydrogen sulfide gas into a solution having dissolved the organic base in the aforesaid solvent in an amount substantially equimolar to said organic base. It is generally preferred to provide the thus prepared solution for the reaction.

As the inorganic salt of hydrogen sulfide, there may effectively be used alkali metal salts, alkaline earth metal salts and ammonium salts of hydrogen sulfide. Typical examples include sodium sulfide, sodium hydrogen sulfide, potassium sulfide, potassium hydrogen sulfide, calcium sulfide, calcium hydrogen sulfide, magensium sulfide, magnesium hydrogen sulfide, ammonium sulfide and ammonium hydrogen sulfide. Such an inorganic salt of hydrogen sulfide may desirably be dissolved in a small amount of water or an alcohol before being provided for the reaction.

It is desirable to use a salt of hydrogen sulfide in an amount of one mole or more, preferably 1.1 to 1.5 mole, per mole of the starting diazoacetonitrile, in view of the yield of the desired product as well as economy.

The contact is carried out under ordinary pressure at a relatively low temperature, i.e., below 20°C. preferably at a temperature of −5 to 15°C., since the starting diazoacetonitrile is liable to be decomposed at a higher temperature.

After completion of the contact, the desired 5-amino-1,2,3-thiadiazole partially precipitates, which is collected by filtration. The resulting filtrate is concentrated to precipitate the remaining desired product from the mother liquor.

Next, Examples of the present invention will be illustrated below.

Example 1

In a reactor was placed 300 g. of a solution of 0.07 mole of diazoacetonitrile in methylene chloride, and the mixture was maintained at around 3°C. and stirred. After 15 g. of triethyl amine was added dropwise thereto over 5 minutes, hydrogen sulfide gas was blown thereto over 30 minutes at a rate of 52 ml/min. Seven minutes after starting of the blowing of hydrogen sulfide gas, crystals of the desired product began to precipitate. After blowing of the hydrogen sulfide gas, the reaction mixture was stirred for 15 minutes and 3.2 g. of the precipitated product was collected by filtration. Subsequently, the filtrate was concentrated under reduced pressure at a temperature of below 10°C. to give 1.4 g. of crystals of the desired product.

Examples 2 to 10

Experiments were run in the same manner as in Example 1 except that the starting materials, solvents, bases and the used amounts thereof were changed to those as shown in the following Table 1. The results are also shown in Table 1.

TABLE 1

| Example No. | Used Starting material | Used solvent | Used base (g.) | Product (g.) |
|---|---|---|---|---|
| 1 | diazoaceto-nitrile | methylene chloride | triethyl amine (15 g.) | 5-amino-1,2,3-thiadiazole (4.6 g.) |
| 2 | diazoaceto-nitrile | methylene chloride | pyridine (11.2 g.) | 5-amino-1,2,3-thiadiazole (3.2 g.) |
| 3 | diazoaceto-nitrile | chloroform | n-butyl amine (10 g.) | 5-amino-1,2,3-thiadiazole (3.4 g.) |
| 4 | diazoaceto-nitrile | ethyl ether | diethyl amine (10 g.) | 5-amino-1,2,3-thiadiazole (1.5 g.) |
| 5 | diazoaceto-nitrile | methylene chloride | hexamethylene-imine (14 g.) | 5-amino-1,2,3-thiadiazole (3.9 g.) |
| 6 | diazoaceto-nitrile | trichloro-ethylene | 2-ethoxy-ethyl amine (12 g.) | 5-amino-1,2,3-thiadiazole (3.2 g.) |
| 7 | diazoaceto-nitrile | 1,2-dichloro-ethane | ethylene-diamine (8 g.) | 5-amino-1,2,3-thiadiazole (2.7 g.) |
| 8 | diazoaceto-nitrile | methyl cellosolve | allyl amine (8 g.) | 5-amino-1,2,3-thiadiazole (2.8 g.) |
| 9 | ethyl-diazoaceto-nitrile | methylene chloride | triethyl amine (15 g.) | 4-ethyl-5-amino-1,2,3-thia-diazole (6.8 g.) |
| 10 | phenyl-diazoaceto-nitrile | methylene chloride | diethyl amine (10 g.) | 4-phenyl-5-amino-1,2,3-thia-diazole (8.9 g.) |

Example 11

In a reactor were placed 200 g. of a solution of 0.077 mole of diazoacetonitrile in methylene chloride and 80 ml. of n-hexane, and the mixture was maintained at around 0°C. and stirred. After 15 g. of triethyl amine was added dropwise thereinto over 15 minutes, hydrogen sulfide gas was blown thereinto over 30 minutes at a rate of 52 ml/min., followed by filtration and concentration, giving 5.3 g. of 5-amino-1,2,3-thiadiazole.

Example 12

In a reactor were placed 200 g. of a solution of 0.083 mole of diazoacetonitrile in methylene chloride and 80 ml. of n-hexane, and the mixture was maintained at around 5°C. and stirred, followed by dropwise addition thereto of 7 g. of triethyl amine over 5 minutes. Subsequently, hydrogen sulfide gas was blown thereinto over 30 minutes at a rate of 28 ml/min., followed by filtration and concentration, giving 6.2 g. of 5-amino-1,2,3-thiadiazole.

Example 13

In a reactor were placed 300 g. of a solution of 0.08 mole of diazoacetonitrile in methylene chloride and 9 g. of diethyl amine, and the mixture was maintained at around 3°C. and stirred. After hydrogen sulfide gas was blown thereinto over 45 minutes at a rate of 52 ml/min., followed by filtration and concentration, giving 4.5 g. of 5-amino-1,2,3-thiadiazole.

Example 14

In a reactor was placed 200 ml. of a solution of 0.07 mole of diazoacetonitrile in ethanol, and the resulting solution was maintained at around —3°C. and stirred. To the stirred solution was added dropwise 100 ml. of a solution of 0.07 mole of anhydrous sodium hydrosulfide in ethanol over 30 minutes, followed by stirring at the same temperature for 15 minutes. As the result, 3.9 g. of 5-amino-1,2,3-thiadiazole was obtained.

Example 15

In a reactor was placed 250 ml. of a solution of 0.07 mole of diazoacetonitrile in methyl alcohol, and the resulting solution was maintained at around —3°C. and stirred. To the stirred solution was added dropwise 20 ml. of a solution of 0.015 mole of sodium methoxide in methyl alcohol over 5 minutes. After hydrogen sulfide gas was blown thereinto over 30 minutes at a rate of 50 ml/min., the reaction mixture was further stirred at the same temperature for 15 minutes.

As the result, 2.9 g. of 5-amino-1,2,3-thiadiazole was obtained.

Example 16

In a reactor was placed 100 ml. of a solution of 0.05 mole of diazoacetonitrile in methylene chloride, and the mixture was maintained at —7°C. and stirred. To the stirred solution was added dropwise 100 ml. of a 50%-ethanolic solution of 3.90 g. (0.05 mole) of anhydrous sodium sulfide ($Na_2S$) over 30 minutes, and then the resulting mixture was further stirred at the same temperature for 15 minutes. As the result, 2.4 g. of 5-amino-1,2,3-thiadiazole was obtained.

Example 17

In a reactor was placed a solution of 4.7 g. (0.07 mole) of diazoacetonitrile in 250 ml. of methyl alcohol. While the solution was maintained at —3°C. under stirring, there was added dropwise a solution of 0.8 g. (0.015 gram equivalent) of sodium methoxide in 20 ml. of methyl alcohol over 5 minutes. After hydrogen sulfide gas was blown thereinto over 30 minutes at a rate of 50 ml/min., the reaction mixture was further stirred for 15 minutes.

The reaction product was subjected to filtration, concentration and extraction to obtain 4.3 g of 5-amino-1,2,3-thiadiazole.

Examples 18—24

Experiments were run in the same manner as in Example 17, except that the starting material, the solvent, the inorganic bases, the amounts thereof and the reaction temperature were changed to those as shown in the following Table 2. The results are also shown in Table 2.

TABLE 2

| Example No. | Starting* material | Solvent | Base | Reaction temperature (°C.) | Product (g.) |
|---|---|---|---|---|---|
| 17 | diazoaceto-nitrile | methyl alcohol | a solution of 0.8 g. (0.015 gram equivalent) of sodium methoxide in 20 ml of methyl alcohol | –3 | 5-amino-1,2,3-thiaziazole (4.3) |
| 18 | diazoaceto-nitrile | ethyl alcohol | a solution of 0.9 g. (0.013 gram equivalent) of sodium ethoxide in 20 ml of ethyl alcohol | –3 | 5-amino-1,2,3-thiaziazole (4.0) |
| 19 | diazoaceto-nitrile | methyl cellosolve | a solution of 1.2 g. (0.02 gram equivalent) of potassium hydroxide in 5 ml. of water | –3 | 5-amino-1,2,3-thiaziazole (3.6) |
| 20 | diazoaceto-nitrile | ethyl alcohol | a solution of 2.1 g. (0.03 gram equivalent) of 28 wt% aqueous ammonia | –3 | 5-amino-1,2,3-thiaziazole (1.7) |
| 21 | diazoaceto-nitrile | ethyl alcohol | a solution of 0.6 g. (0.01 gram equivalent) of barium hydroxide in 50 ml. of water | 5 | 5-amino-1,2,3-thiaziazole (1.8) |

TABLE 2 (cont'd)

| Example No. | Starting* material | Solvent | Base | Reaction temperature (°C.) | Product (g.) |
|---|---|---|---|---|---|
| 22 | diazoaceto-nitrile | ethyl-alcohol | a solution of 2.2 g. (0.02 gram equivalent) of sodium carbonate in 10 ml. of water | 5 | 5-amino-1,2,3-thiaziazole (2.3) |
| 23 | ethyldiazo-acetonitrile | methyl alcohol | the same as in Example 17 | –3 | 4-ethyl-5-amino-1,2,3-thiadiazole (5.2) |
| 24 | phenyldiazo-acetonitrile | methyl alcohol | the same as in Example 17 | –3 | 4-phenyl-5-amino-1,2,3-thiadiazole (6.5) |

* Each used in an amount of 0.07 mole.

Example 25

In a reactor was placed a solution of 4.7 g (0.07 mole) of diazoacetonitrile in 250 ml. of dioxane. While the solution was maintained at 0°C. under stirring, there was added a solution of 0.84 g. (0.02 mole) of sodium hydroxide in 5 ml. of water. After hydrogen sulfide gas was blown thereinto over 30 minutes at a rate of 50 ml/min., the reaction mixture was further stirred for 15 minutes.

The reaction product was subjected to filtration, concentration and extraction to obtain 3.7 g. of 5-amino-1,2,3-thiadiazole.

Examples 26—33

Into solutions of various organic bases dissolved in predetermined amounts of 50 ml. of various solvents were blown hydrogen sulfide gas in amounts substantially equimolar to said organic bases to prepare solutions of various hydrogen sulfide salts.

A solution of 4.7 g. (0.07 mole) of diazoacetonitrile in 200 ml. of methylene chloride was placed in a reactor. While the solution was maintained at around —3°C. under stirring, there was added dropwise each hydrogen sulfide salt solution as prepared above over 30 minutes. The reaction mixture was further stirred for 10 minutes.

The reaction product was subjected to filtration, condensation and extraction to obtain 5-amino-1,2,3-thiadiazole.

The solvents, the organic bases, the amounts thereof used in the preparation of the hydrogen sulfide salt solution are shown in the following Table 3. The yields of 5-amino-1,2,3-thiadiazole are also shown in Table 3.

TABLE 3

| Example No. | Preparation of hydrogen sulfide salt | | Yield of 5-amino-1,2,3-thiadiazole (g.) |
|---|---|---|---|
| | Solvent | Organic base (Amount, g.) | |
| 26 | methylene chloride | triethylamine (7.1) | 4.9 |
| 27 | carbon tetrachloride | methylamine (2.2) | 3.1 |
| 28 | methylene chloride | diethylamine (5.1) | 2.5 |
| 29 | chloroform | trimethyl-amine (4.1) | 4.6 |
| 30 | carbon tetrachloride | allylamine (1.9) | 2.8 |
| 31 | methylene chloride | 2-ethoxyethyl-amine (6.2) | 2.9 |
| 32 | methylene chloride | ethylene diamine (2.1) | 2.2 |
| 33 | chloroform | pyridine (5.5) | 3.2 |

Example 34

An experiment was run in the same manner as in Example 26, except that 6.6 g. (0.07 mole) of ethyldiazoacetonitrile was used instead of diazoacetonitrile.

As the result, 6.3 g. of 4-ethyl-5-amino-1,2,3-thiadiazole was obtained.

Example 35

An experiment was run in the same manner as in Example 26, except that 10.0 g. (0.07 mole) of phenyldiazoacetonitrile was used instead of diazoacetonitrile.

As the result, 8.6 g. of 4-phenyl-5-amino-1,2,3-thiadiazole was obtained.

Example 36

In a reactor was placed a solution of 4.7 g. (0.07 mole) of diazoacetonitrile in 300 ml. of carbon tetrachloride. While the solution was maintained at around 5°C. under stirring, there was added dropwise a solution of 5.5 g. (0.07 mole) of anhydrous sodium sulfide in 50 wt% ethyl alcohol over 45 minutes. Then, the reaction mixture was further stirred for 10 minutes.

The reaction product was subjected to filtration, concentration and extraction to obtain 5-amino-1,2,3-thiadiazole.

Examples 37—41

Experiments were run in the same manner as in Example 36, except that aqueous solution of various inorganic salts of hydrogen sulfide were used instead of the solution of anhydrous sodium sulfide in ethyl alcohol. The quantities of water used to dissolve the inorganic salts were 5 ml. in Examples 37 and 38, 10 ml. in Examples 39 and 40, and 15 ml. in Example 41. The inorganic salts of hydrogen sulfide employed, the quantities thereof and the yields of 5-amino-1,2,3-thiadiazole are shown in the following Table 4.

TABLE 4

| Example No. | Inorganic salt of hydrogen sulfide (g.) | Yield of 5-amino-1,2,3-thiadiazole (g.) |
|---|---|---|
| 36 | anhydrous sodium sulfide (5.5) | 2.8 |
| 37 | ammonium sulfide (6.0) | 4.1 |
| 38 | ammonium hydrogen sulfide (3.6) | 4.3 |
| 39 | potassium sulfide (7.7) | 2.3 |
| 40 | potassium hydrogen sulfide (5.0) | 2.1 |
| 41 | calcium hydrogen sulfide (3.7) | 1.9 |

Example 42

In a reactor was placed a solution of 4.7 g. (0.07 mole) of diazoacetonitrile in 200 ml. of methylene chloride. While the solution was maintained at about 3°C. under stirring, there was added dropwise 10 ml. of a solution of 4.1 g. (0.07 mole) of anhydrous sodium hydrogen sulfide in 10 ml. of water over 30 minutes. The reaction mixture was further stirred for 10 minutes. The reaction product was subjected to filtration, concentration and extraction to obtain 4.1 g. of 5-amino-1,2,3-thiadiazole.

Example 43

An experiment was run in the same manner as in Example 42, except that 6.6 g. (0.07 mole) of ethyldiazoacetonitrile was used instead of diazoacetonitrile.

As the result, 6.0 g. of 4-ethyl-5-amino-1,2,3-thiadiazole was obtained.

Example 44

An experiment was run in the same manner as in Example 42, except that 10.0 g. (0.07 mole) of phenyl-diazoacetonitrile was used instead of diazoacetonitrile.

As the result, 8.1 g. of 4-phenyl-5-amino-1,2,3-thiadiazole was obtained.

## Claims

1. A process for preparing a 5-amino-1,2,3-thiadiazole represented by the formula

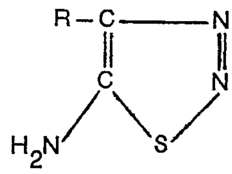

wherein R represents a hydrogen atom, a $C_1$—$C_6$ alkyl group or an aryl group, characterized in that a diazoacetonitrile represented by the formula

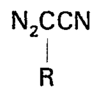

wherein R has the same meaning as defined above, is brought into contact with hydrogen sulfide in the presence of a base or with a salt of hydrogen sulfide, in a solvent.

2. A process according to Claim 1 wherein said solvent is methylene chloride, diethyl ether, chloroform or n-hexane.

3. A process according to Claim 1 wherein said base is an organic base.

4. A process according to Claim 3 wherein said base is an amine series compound.

5. A process according to Claim 4 wherein said amine series compound is dimethyl amine, diethyl amine, dibutyl amine, trimethyl amine, triethyl amine or tributyl amine.

6. A process according to Claim 1, wherein said base is an inorganic base.

7. A process according to Claim 6 wherein said base is sodium hydroxide, potassium hydroxide, ammonia, sodium methoxide or sodium ethoxide.

8. A process according to Claim 1, wherein said salt of hydrogen sulfide is a salt between hydrogen sulfide and an organic base.

9. A process according to Claim 1, wherein said salt of hydrogen sulfide is a salt between hydrogen sulfide and an inorganic base.

10. A process according to Claim 1 wherein said reaction is carried out at a temperature of −5 to 15°C.

## Revendications

1. Procédé pour la préparation d'un 5-amino-1,2,3-thiadiazole représenté par la formule:

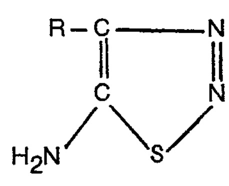

dans laquelle R représente un atome d'hydrogène, un groupe alkyle en $C_1$—$C_6$ ou un groupe aryle, caractérisé par le fait qu'un diazoacétonitrile représenté par la formule:

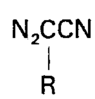

dans laquelle R a la même définition que ci-dessus, est amené en contact avec de l'hydrogène sulfuré en présence d'une base, ou bien avec un sel de l'hydrogène sulfuré, dans un solvant.

2. Procédé selon la revendication 1, dans lequel ledit solvant est le chlorure de méthylène, l'éther diéthylique, le chloroforme ou le n-hexane.

3. Procédé selon la revendication 1, dans lequel ladite base est une base organique.

4. Procédé selon la revendication 3, dans laquelle ladite base est un composé de la famille des amines.

5. Procédé selon la revendication 4, dans lequel ledit composé de la famille des amines est la diméthylamine, la diéthylamine, la dibutylamine, la triméthylamine, la triéthylamine ou la tributylamine.

6. Procédé selon la revendication 1, dans lequel ladite base est une base minérale.

7. Procédé selon la revendication 6, dans lequel ladite base est l'hydroxyde de sodium, l'hydroxyde de potassium, l'ammoniaque, le méthylate de sodium ou l'éthylate de sodium.

8. Procédé selon la revendication 1, dans lequel ledit sel de l'hydrogène sulfuré est un sel résultant de la réaction entre l'hydrogène sulfuré et une base organique.

9. Procédé selon la revendication 1, dans lequel ledit sel de l'hydrogène sulfuré est un sel résultant de la réaction entre l'hydrogène sulfuré et une base minérale.

10. Procédé selon la revendication 1, dans lequel ladite réaction est effectuée à une température de $-5$ à $+15$°C.

**Patentansprüche**

1. Verfahren zur Herstellung eines 5-Amino-1,2,3-thiadiazols der Formel

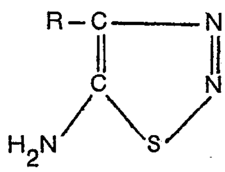

worin R ein Wasserstoffatom, eine $C_1$—$C_6$ Alkylgruppe oder eine Arylgruppe bedeutet, dadurch gekennzeichnet, daß man ein Diazoacetonitril der Formel

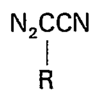

worin R die vorher angegebene Bedeutung hat in einem Lösungsmittel in Berührung mit Schwefelwasserstoff in Gegenwart einer Base oder mit einem Schwefelwasserstoffsalz bringt.

2. Verfahren gemäß Anspruch 1, dadurch gekennzeichnet, daß das Lösungsmittel Methylenchlorid, Diethylether, Chloroform oder n-Hexan ist.

3. Verfahren gemäß Anspruch 1, dadurch gekennzeichnet, daß die Base eine organische Base ist.

4. Verfahren gemäß Anspruch 3, dadurch gekennzeichnet, daß die Base eine Aminverbindung ist.

5. Verfahren gemäß Anspruch 4, dadurch gekennzeichnet, daß die Aminverbindung Dimethylamin, Diethylamin, Dibutylamin, Trimethylamin, Triethylamin oder Tributylamin ist.

6. Verfahren gemäß Anspruch 1, dadurch gekennzeichnet, daß die Base eine anorganische Base ist.

7. Verfahren gemäß Anspruch 6, dadurch gekennzeichnet, daß die Base Natriumhydroxid, Kaliumhydroxid, Ammoniak, Natriummethoxid oder Natriumethoxid ist.

8. Verfahren gemäß Anspruch 1, dadurch gekennzeichnet, daß das Schwefelwasserstoffsalz ein Salz aus Schwefelwasserstoff und einer organischen Base ist.

9. Verfahren gemäß Anspruch 1, dadurch gekennzeichnet, daß das Schwefelwasserstoffsalz ein Salz zwischen Schwefelwasserstoff und einer anorganischen Base ist.

10. Verfahren gemäß Anspruch 1, dadurch gekennzeichnet, daß die Umsetzung bei einer Temperatur von $-5$ bis $-15$°C durchgeführt wird.